# EUROPEAN PATENT APPLICATION

(11) **EP 3 181 220 A1**
(43) Date of publication of application: **21.06.2017**
(21) Application number: 15200517.9
(22) Date of filing: 16.12.2015
(51) Int. Cl.: B01J 8/00, B03C 1/015, C07C 209/48

(54) **A PROCESS FOR REMOVING A HETEROGENEOUS CATALYST FROM A REACTION PRODUCT AND A PROCESS FOR PRODUCING AN AROMATIC AMINE**

(71) Applicant: BASF SE, 67056 Ludwigshafen am Rhein (DE)
(72) Inventor: DAI, Yuan Shen, 200037 Shanghai (CN); RENZE, Peter, 68163 Mannheim (DE)
(74) Representative: Kudla, Karsten

(57) **Abstract**

A process for removing a heterogeneous catalyst from a reaction product and a process for producing an aromatic amine

Abstract

The invention relates to a process for removing a heterogeneous catalyst from a crude reaction product (9) which comprises a liquid phase and a particulate catalyst, comprising at least one of the following steps:
(a) feeding the crude reaction product (9) into a hydrocyclone (11) in which at least part of the particulate catalyst is removed and taking off a product stream (13) depleted in catalyst and a stream (15) enriched in catalyst;
(b) feeding the crude reaction product (9) or, when step (a) also is carried out, the product stream (13) depleted in catalyst of step (a) into a settler (17), the settler (17) comprising a conical bottom part (19) wherein nozzles (27) are arranged in the conical bottom part (19) such that by a stream fed through the nozzles (27) a swirl is generated in the conical bottom part (19) of the settler (17), and taking off a product stream (29) and a catalyst comprising stream (21).

The invention further relates to a process for producing an aromatic amine in which catalyst is removed from the crude reaction product (9) by the above process.

## Description

The invention relates to a process for removing a heterogeneous catalyst from a reaction product which comprises a liquid phase and a particulate catalyst. The invention further relates to a process for producing an aromatic amine in which a nitro compound, hydrogen and a catalyst are fed into a reactor and the nitro compound and the hydrogen react in presence of the catalyst forming the aromatic amine.

Aromatic amines, particularly toluenediamine, are frequently used in industry. They are especially used as reactant in the production of the corresponding diisocyanates which find use in the preparation of polyurethanes.

Aromatic amines generally are produced by hydrogenation of nitro compounds. A process for producing aromatic amines is disclosed for example in US 6,350,911. The reaction is carried out in a vertical reactor which comprises a downward-facing jet nozzle arranged in the upper part of the reactor. Through the jet nozzle starting materials and a part of the reaction mixture which is recycled in an external loop are fed in. The vertical reactor is a loop reactor in which the reaction mixture comprising a liquid phase, a gas phase and a solid phase form a loop flow. At the bottom of the reactor crude reaction product is discharged. The reaction product is fed to a purification in which the aromatic amine is separated off.

A process for producing toluenediamine by hydrogenating dinitrotoluene is described for example in US-A 2011/275858. The reactor corresponds to that as disclosed in US 6,350,911. The product stream flows through a crossflow filter to remove catalyst. The catalyst is recycled in the reactor and the product stream depleted of catalyst is taken off the process.

Further processes for producing aromatic amines in which a filter unit, for example a crossflow filter, is used to remove catalyst are disclosed in US-A 2014/020689 or US 5,563,296.

Besides using a filter which can be blocked by catalyst particles it is known to use a settler for separating the solid catalyst from the crude reaction product. The settler comprises a conical bottom part through which the catalyst is discharged and recycled into the reactor. As the catalyst tends to deactivate, it is necessary to replace catalyst during the process. This is realized by removing a part of the catalyst and replace by new catalyst. However, in the conical part of the settler a layer of catalyst accumulates which has to be removed periodically.

Therefore, it has been an object of the present invention to provide a process for removing a heterogeneous catalyst from a reaction product which has not the disadvantages of the processes as known in the art. Particularly, a frequent removal of catalyst should be avoided.

This object is achieved by a process for removing a heterogeneous catalyst from a crude reaction product which comprises a liquid phase and a particulate catalyst, comprising at least one of the following steps:
(a) feeding the crude reaction product into a hydrocyclone in which at least part of the particulate catalyst is removed and taking off a product stream depleted of catalyst and a stream enriched in catalyst;
(b) feeding the crude reaction product or, when step (a) also is carried out, the product stream depleted of catalyst into a settler, the settler comprising a conical bottom part wherein nozzles are arranged in the conical bottom part such that by a stream fed through the nozzles a swirl is generated in the conical bottom part of the settler, and taking off a product stream and a catalyst comprising stream.

By feeding the crude reaction product into a hydrocyclone, particulate catalyst is removed. It is an advantage of a hydrocyclone that no fouling by precipitation of catalyst occurs. In the hydrocyclone a majority of the catalyst is separated from the crude reaction product. However, the product stream depleted of catalyst generally still contains a small amount of catalyst. This catalyst can be removed for example in a filter system or in a settler. When the hydrocyclone is used, it is not mandatory to provide nozzles in the conical part of the settler. However, it also is possible to feed the product stream depleted of catalyst which is taken off the hydrocyclone into a settler comprising a conical bottom part wherein nozzles are arranged in the conical part.

The hydrocyclone which is used in connection with the present invention preferably is dimensioned such that a defined cut size is reached. The cut size is the particle size for which 50% of the particles leave the hydrocyclone through the overflow (product stream depleted of catalyst) and 50% of the particles leave the hydrocyclone through the underflow (product stream enriched in catalyst). The cut size preferably is in the range from 5 to 30 µm. Depending on the crude product flow rate to be processed it is possible to use one or more hydrocyclones. When more than one hydrocyclone is used, the hydrocyclones are connected in parallel.

The crude reaction product containing abrasive catalyst particles can cause abrasion of the hydrocyclone interior, which can negatively influence the separation characteristics, e.g. cut size. Therefore, in a preferred embodiment the hydrocyclone is designed such that the vortex pipe or the apex or both the vortex pipe and the apex can be exchanged quickly with preferably little effort.

When only a settler is used, a settler having nozzles arranged in the conical bottom part is used. By the stream fed into the settler through the nozzles a swirl is generated in the bottom part of the settler. This surprisingly reduces the forming of a catalyst layer in the bottom part of the settler.

In a preferred embodiment the nozzles are installed essentially at the same height in the conical bottom part of the settler. Independently of the number of nozzles, the nozzles are preferably arranged with constant distances between the nozzles. In a particularly preferred embodiment two nozzles are arranged at the same height in the conical bottom part of the settler with opposite direction with an angle in the range from 150 to 210°, preferably with an angle in the range from 165 to 195° and particularly preferable with an angle in the range from 175 to 185°. Essentially at the same height means that the angle between the line which connects two nozzles and the horizontal line is in the range from -10 to 10°, preferably in the range from -5 to 5° and particularly preferably in the range from -2 to 2°.

Depending on the total height of the conical bottom part of the settler it is also possible to install nozzles at different horizontal levels of the conical bottom part. In this case on each level at least two nozzles are installed. Particularly preferable is the installation of two nozzles on each horizontal level. The arrangement of the nozzles on each level thereby corresponds to the arrangement of nozzles when they are installed at only one level.

The nozzles which are installed in the conical bottom part of the settler preferably are designed such that the spray velocity of the nozzle is in the range from 0.1 to 1 m/s. The nozzle diameter preferably is in the range from 25 to 100 mm, particularly preferably in the range from 35 to 50 mm.

The settler generally is designed such that at the top a gaseous stream can be taken off and the catalyst comprising stream is taken off at the conical part of the settler. However, the gaseous stream only needs to be taken off in case there is no other option to remove the gas. Above the conical part generally there is a part having a constant cross sectional area. In this part the reaction product or when the reaction product first has been fed into the hydrocyclone the product stream depleted of catalyst leaving the hydrocyclone is fed in and the product stream is taken off.

In order to avoid feeding additional material into the settler through the nozzles, it is preferred, that part of the catalyst comprising stream which is taken off the settler is fed into the settler through the nozzles. By feeding part of the catalyst comprising stream which is taken off the settler, no additional material needs to be used. A further advantage is that only components are added into the settler which already are in the settler. Additionally, the composition corresponds to that of the material which is in the settler. Particularly in case of an equilibrium reaction this has the advantage that the equilibrium is not shifted and the reaction will not continue in the settler.

Particularly in a continuous process, it is preferred to feed the part of the catalyst comprising stream which is not fed into the settler through the nozzles into a reactor from which the reaction product results. In this way, the catalyst can be reused and it is not necessary to replace all of the catalyst which is taken off the reactor by fresh catalyst. Further, reactants which are not converted into the aromatic amine also are fed back into the reactor and can react. Hereby the conversion can be improved. It does not matter that also reaction product may be in the stream. This has the additional advantage that the reaction product is not removed together with used catalyst but can be taken off later with the product stream.

To gain the liquid part of the catalyst comprising stream as additional product stream, in a preferred embodiment the stream enriched in catalyst which is taken off the hydrocyclone is fed into a catalyst discharge system from which a stream comprising substantially catalyst and a stream being essentially free of catalyst are taken off. It also is possible to feed the catalyst comprising stream which is taken off the settler into the catalyst discharge system. In the catalyst discharge system the catalyst is separated from the liquids in the stream. A part of the catalyst preferably is recycled into the reactor and the rest of the catalyst is removed and replaced by fresh catalyst. However, it is also possible to remove all of the catalyst separated off in the catalyst discharge system and replace it by fresh catalyst to keep the amount of catalyst in the process constant. The stream which is essentially free of catalyst generally comprises reactants and is, therefore, fed back into the reactor from which the reaction product results.

Suitable catalyst discharge systems are for example filters. Particularly preferably the catalyst discharge system is selected from the group consisting of candle filters, cross flow filters and magnetic separators.

Gas which may be comprised in the reaction product and which should be removed can be removed for example in the settler. To remove the gas in the settler, a gas venting preferably is placed on top of the settler.

In a particularly preferred embodiment, the liquid phase of the reaction product comprises an aromatic amine. The aromatic amine preferably is toluenediamine or aniline.

The invention further relates to a process for producing an aromatic amine, comprising following steps:
(i) feeding of a nitro compound, hydrogen and catalyst as starting materials into a reactor;
(ii) reacting the nitro compound and the hydrogen in presence of the catalyst forming the aromatic amine;
(iii) taking off a crude reaction product comprising aromatic amine and catalyst from the reactor;
(iv) feeding the crude reaction product into a process for removing heterogeneous catalyst as outlined above.

The conversion of the starting materials forming the aromatic amine for example is carried out as disclosed in US 6,350,911. In this process, the reactor preferably is a vertical, preferably cylindrical reactor whose length is greater than its diameter, having a downward-facing jet nozzle arranged in the upper region of the reactor through which the starting materials and recirculated reaction mixture are fed in, and having an outlet at any desired point of the reactor, preferably in the lower region, through which the reaction mixture is fed back to the jet nozzle in an external circuit by means of a conveying means, preferably a pump, and having flow reversal in the lower region of the reactor. The reactor described is referred to as a loop reactor.

The flow reversal and thus the formation of internal loop flow can be effected, in the case of take-off of the reaction mixture in the upper region of the reactor, by impact of the injected reaction mixture on the reactor base. In the case of the preferred take-off of the reaction mixture in the lower region of the reactor, the flow reversal is achieved by internals, in particular a baffle plate perpendicular to the reactor wall.

In a preferred embodiment of the novel process, the loop reactor has a preferably concentric spigot parallel to the reactor wall between the nozzle and the flow reversal. This spigot can be in the form of a simple tube, a tubular plate heat exchanger or a coiled cooling tube.

The concentric spigot in combination with the baffle plate stabilizes the loop flow within the reactor, referred to below as internal loop flow. Besides producing flow reversal, the baffle plate makes sure that no gas bubbles are dragged into the external loop flow and damage the pump.

The fact that the diameter of the loop reactor is smaller than its height produces adequate loop flow throughout the reactor and prevents the formation of dead zones.

In a further preferred embodiment of the invention, the reactor has integrated heat exchangers in the reactor interior. These heat exchangers should be of such a design that they do not prevent internal loop flow. Examples of heat exchangers which can be used are tubes carrying cooling medium, which are preferably parallel to the reactor wall, plate heat exchangers, which are preferably parallel to the reactor wall, or boiling tubes closed at the bottom, as described in EP-A 263 935, known as Field tubes. If Field tubes are used, it is possible to utilize the steam formed as process steam.

In this embodiment, the reactor used in accordance with the invention can be regarded as a reaction heat exchanger since the heat of reaction is dissipated at the point where it is formed. It is also possible to install a heat exchanger in the external loop flow in addition to the heat exchangers integrated in the reactor.

The majority of the reaction mixture is transported in the internal loop flow; only a small proportion of the reaction mixture is pumped externally, thus providing the drive for the loop flow. The ratio between the volume flow rates of the internal loop flow and the external loop flow is from 2:1 to 30:1, preferably from 5:1 to 10:1.

The nitro compound which is fed into the reactor is a mono- and/or polynitro compound which is employed in pure form, as a mixture with the corresponding mono- and/or polyamine, as a mixture with the corresponding mono- and/or polyamine and water or as a mixture with the corresponding mono- and/or polyamine, water and a solvent, in particular an alcoholic solvent. The aromatic mono and/or polynitro compound is introduced into the mixture in finely divided form. The nitro compound is preferably introduced into the jet nozzle, particularly preferably into the mixing chamber of the nozzle.

Use is preferably made of aromatic nitro compounds having one or more nitro groups and 6 to18 carbon atoms, for example nitrobenzenes, for example nitrobenzene, 1,3-dinitrobenzene, nitrotoluenes, for example 2,4 or 2,6-dinitrotoluene, 2,4,6-trinitrotoluene, nitroxylenes, for example 1,2-dimethyl-3-, 1,2-dimethyl-4-, 1,4-dimethyl-2-, 1,3-dimethyl-2-, 2,4-dimethyl-1- and 1,3-dimethyl-5-nitrobenzene, nitronaphthalenes, for example 1-, 2-nitronaphthalene, 1,5 and 1,8-dinitronaphthalene, chloronitrobenzenes, for example 2-chloro-1,3- and 1-chloro-2,4-dinitro-benzene, o-, m-and p-chloronitrobenzene, 1,2-dichloro-4-, 1,4-dichloro-2-, 2,4-dichloro-1-and 1,2-dichloro-3-nitrobenzene, chloronitrotoluenes, for example 4-chloro-2-, 4-chloro-3-, 2-chloro-4-, and 2-chloro-6-nitrotoluene, nitroanilines, for example o-, m- and p-nitroaniline; nitroalcohols, for example tris(hydroxymethyl)-nitromethane, 2-nitro-2-methyl- and 2-nitro-2-ethyl-1,3-propanediol, 2-nitro-1-butanol and 2-nitro-2-methyl-1-propanol, and any desired mixtures of two or more of said nitro compounds.

The process preferably is used to hydrogenate aromatic nitro compounds, preferably mono-nitrobenzene, methylnitrobenzene or methylnitrotoluene, and in particular 2,4-dinitrotoluene or technical-grade mixtures thereof with 2,6-dinitrotoluene, where these mixtures preferably contain up to 35% by weight, based on the total mixture, of 2,6-dinitrotoluene with proportions of from 1 to 4 percent of vicinal dinitrotoluene and from 0.5 to 1.5% of 2,5- and 3,5-dinitrotoluene, to the corresponding amines.

Catalysts which can be used in the process can be all heterogeneous hydrogenation catalysts known per se for aromatic nitro compounds. The heterogeneous catalysts are employed in the finely divided state and are suspended in the reaction suspension in the finely divided form. Suitable catalysts are metals from sub-group VIII of the Periodic Table, which may be supported on support materials such as activated carbon or oxides of aluminum, silicon or other materials.

Preference is given to Raney nickel and/or supported catalysts based on nickel, palladium and/or platinum.

Particularly advantageous in the process is the use of a platinum/iron catalyst supported on carbon. The carbon support employed is, in particular, a hydrophobic carbon. Activated carbon or acetylene black is preferably used. The platinum is usually on the support in the form of the metal. The iron is usually in the form of an iron compound, preferably as iron (III)oxide. The platinum is usually present in an amount in the range from 0.1 to 10% by weight, based on the weight of the support, and the iron is usually present in an amount in the range from 0.1 to 10% by weight, calculated as iron(III)oxide and likewise based on the support.

Irrespective of the type of the nitro compounds employed, it is preferred to maintain a pressure in the range from 5 to 100 bar, preferably from 10 to 50 bar, and an operating temperature of from 80 to 200°C, preferably from 100 to 150°C in the reactor.

The product is discharged as crude reaction product from the reactor continuously at any desired point, but preferably in the lower region of the reactor at its base or in particular from the external loop flow. This crude reaction product then is fed into the process for removing the heterogeneous catalyst as described above.

An embodiment of the invention is shown in the figure and described below.

The only figure shows a flow chart of a process for producing aromatic amines.

A nitro compound 1, hydrogen 3 and a solid catalyst 5 are fed into a reactor 7. The reactor preferably is a loop reactor. In the reactor an aromatic amine is formed by reaction of the nitro compound and the hydrogen in presence of the solid catalyst. From the reactor 7 a crude reaction product 9 is taken off.

The crude reaction product 9 preferably has a temperature in the range from 80 to 200°C, particularly in the range from 100 to 150°C and a pressure in the range from 5 to 100 bar, preferably in the range from 10 to 50 bar. The amount of solid catalyst in the crude reaction product preferably is in the range from 0.01 to 20 wt% and particularly in the range from 0.1 to 15 wt%. The average size of the catalyst particles generally is in the range from 0.01 to 200 µm and particularly in the range from 0.01 to 100 µm.

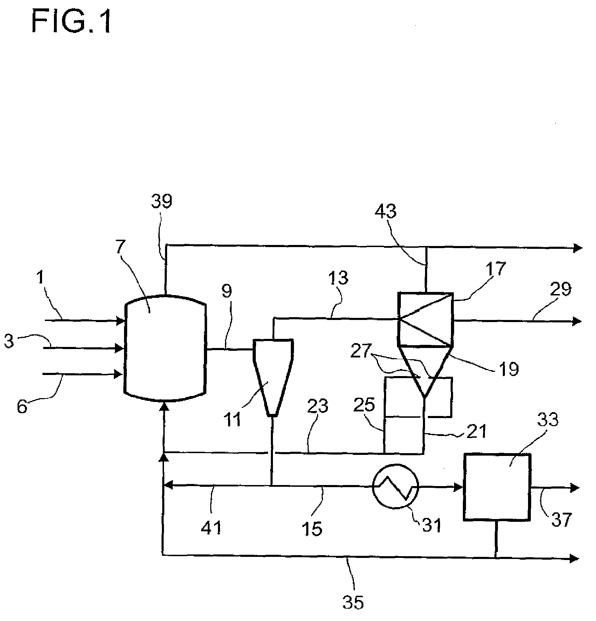

In the embodiment as shown in figure 1, the crude reaction product 9 is fed into a hydrocyclone 11 in which at least a part of the solid catalyst is removed. At the top of the hydrocyclone 11 a stream 13 depleted of catalyst is taken off and at the bottom of the hydrocyclone 11 a stream 15 enriched in catalyst is taken off.

The stream 13 depleted in catalyst preferably has a temperature in the range from 80 to 200°C, particularly in the range from 100 to 150°C and a pressure in the range from 5 to 100 bar, preferably in the range from 10 to 50 bar. The amount of solid catalyst in the stream 13 depleted in catalyst is in the range from 0.001 to 10 wt% and particularly in the range from 0.01 to 5 wt%. The average size of the catalyst particles is in the range from 0.01 to 30 µm and particularly in the range from 0.01 to 10 µm.

The stream 15 enriched in catalyst preferably also has a temperature in the range from 80 to 200°C, particularly in the range from 100 to 150°C and a pressure in the range from 5 to 100 bar, preferably in the range from 10 to 50 bar. The amount of catalyst preferably is in the range from 0.01 to 40 wt% and particularly in the range from 1 to 40 wt%. The average size of the catalyst particles generally is in the range from 0.1 to 200 µm and particularly in the range from 5 to 100 µm.

In the embodiment of figure 1, the stream 13 depleted in catalyst is fed into a settler 17. In the settler 17 additional catalyst is separated from the reaction product. The settler comprises a conical bottom part 19 in which a catalyst comprising stream 21 is collected and taken off. The catalyst comprising stream 21 is divided into two partial streams 23, 25, a first partial stream 23 being recycled into the reactor 7 and a second partial stream 25 being recycled into conical bottom part 19 of the settler 17 via nozzles 27. The nozzles 27 are arranged such that a swirl is formed in the conical bottom 19 part of the settler 17. The swirl is formed for example by setting the nozzles 27 in tangential direction. As described above, preferably at least two nozzles are placed in the substantially same horizontal height of the conical bottom part 19 of the settler 17.

The catalyst comprising stream 21 and therefore also the first partial stream 23 and the second partial stream 25 preferably have a temperature in the range from 80 to 200°C, particularly in the range from 100 to 150°C and a pressure in the range from 5 to 100 bar, preferably in the range from 10 to 50 bar. The amount of catalyst preferably is in the range from 0.01 to 40 wt% and particularly in the range from 5 to 40 wt%. The average size of the catalyst particles generally is in the range from 0.1 to 200 µm and particularly in the range from 5 to 100 µm.

From the upper part of the settler 17 a product stream 29 substantially free of catalyst is removed. The product stream 29 has a temperature in the range from 80 to 200°C, particularly in the range from 100 to 150°C and a pressure in the range from 5 to 100 bar, preferably in the range from 10 to 50 bar. The amount of catalyst preferably is in the range from 0.001 to 5 wt% and particularly in the range from 0.001 to 1 wt%. The average size of the catalyst particles generally is in the range from 0.01 to 30 µm and particularly in the range from 0.01 to 5 µm.

The stream 15 enriched in catalyst which is taken off the hydrocyclone 11 is cooled down to a temperature in the range from 80 to 150°C, preferably in the range from 80 to 100°C in a cooler 31 and afterwards fed into a catalyst discharge system 33. In the cooler, additionally the pressure is reduced to a pressure in the range from 0 to 50 bar, preferably in the range from 0 to 10 bar.

Further, a return line 41 is provided which branches off the line through which the stream 15 enriched in catalyst flows into the cooler 31. Through the return line 41 at least part of the stream 15 enriched in catalyst can be recycled into the reactor 7.

As described above, the catalyst discharge system 33 preferably is a separator, for example a candle filter, a cross flow filter or a magnetic separator. In the catalyst discharge system 33 the stream 15 enriched in catalyst is separated into a stream 35 substantially free of catalyst and a stream 37 substantially consisting of catalyst. A part of the stream 35 substantially free of catalyst is recycled back into the reactor and a second part of the stream 35 substantially free of catalyst is removed from the process. The stream 37 substantially consisting of catalyst also is removed from the process.

In the line through which the stream 35 substantially free of catalyst is recycled into the reactor 7, a preheater and/or a pump can be optionally installed.

The stream 35 substantially free of catalyst has a temperature in the range from 50 to 150°C, particularly in the range from 80 to 100°C and a pressure in the range from 0 to 50 bar, preferably in the range from 0 to 10 bar. The amount of catalyst preferably is in the range from 0.001 to 5 wt% and particularly in the range from 0.001 to 1 wt%. The average size of the catalyst particles generally is in the range from 0.01 to 30 µm and particularly in the range from 0.01 to 5 µm.

The stream 37 substantially consisting of catalyst has a temperature in the range from 10 to 150°C, particularly in the range from 10 to 100°C and a pressure in the range from 0 to 50 bar, preferably in the range from 0 to 10 bar. The amount of catalyst preferably is in the range from 40 to 95 wt% and particularly in the range from 60 to 95 wt%. The average size of the catalyst particles generally is in the range from 0.01 to 200 µm.

In all streams 9, 13, 15, 21, 23, 25, 29, 35 and 37 the chemical composition of the liquid phase is similar. The aromatic amine, preferably TDA, is in the range from 40 to 80 wt%, preferably 55 to 65 wt%, based on the total amount of gas and liquid components. Gases can be comprised in an amount from 0.01 to 2 wt%, preferably from 0.05 to 0.5 wt%, also based on the total amount of gas and liquid components. Gases are for example hydrogen, ammonia, nitrogen, oxygen or any suitable mixture of two or more of these gases. The rest of the liquid phase is solvent and/or water. The solvent for example is an alcoholic solvent.

To remove gases from the process, at least one gas outlet is provided. This gas outlet can be provided in the reactor 7 or the settler 17. In the embodiment as shown in the figure, a first gas outlet 39 is provided at the top of the reactor 7, and a second gas outlet 43 at the top of the settler 17.

Besides the embodiment as shown in the figure, it is also possible to omit the hydrocyclone. In this case, the crude reaction product 9 directly is fed into the settler 17. In another embodiment, the settler 17 is omitted, which means that the stream 13 depleted in catalyst which is taken off the hydrocyclone 11 is the product stream.

When an hydrocyclone 11 and a settler 17 are used, it is possible to use a settler without nozzles 27. However, in a particular preferred embodiment the crude product stream 9 is treated in a process as shown in the figure.

### Examples

### Comparative Example

A crude reaction product comprising 1 to 20 wt% of solid catalyst is fed into a settler, wherein there are no nozzles in the bottom part of the settler. The product stream which is taken off the settler comprises 0.3 wt% catalyst with an average size of 10 µm.

In the conical bottom part of the settler, a catalyst layer accumulates with a thickness increasing speed of about 1 mm/month. From time to time the catalyst layer suddenly collapses and falls down to the bottom outlet of the settler and is fed back into the reactor, causing fluctuations in the reactor system.

### Example 1

In the setup of the comparative example a hydrocyclone is added between the reactor and the settler. The hydrocyclone has a design with a cut size of 5 µm. Due to this design all catalyst particles with a diameter of less than 5 µm are captured by the hydrocyclone and fed into a catalyst discharge system.

To treat the whole stream of crude reaction product, a system of 3 hydrocyclones set in parallel has been used. The hydrocyclones have a shared feed and shared outlets for catalyst enriched stream and the stream depleted in catalyst. The stream depleted in catalyst is fed into the settler.

The catalyst discharge system is operated batchwise in such a way that catalyst is removed in such an amount that the catalyst concentration in the reactor remains in an amount of less than 10 wt%.

The crude reaction product fed into the hydrocyclone has a maximum catalyst concentration of 1 to 10 wt%. The product stream taken off the settler comprises 0.03 wt% of catalyst with an average size of 2 µm. In the conical bottom part of the settler a catalyst layer accumulates with a thickness increasing speed of 0.5 mm/month.

### Example 2

The process of the comparative example has been repeated with the difference that the settler has been designed with nozzles in the conical bottom part. In the settler, two nozzles have been installed horizontal with an angle of 0°. The nozzles have been opposite to one another with an angle of 180°. The spray direction of the nozzles is tangential to the circumference of the conical bottom part. The pair of nozzles has been installed in the upper part of the conical bottom part at 1/10 of the height of the conical bottom part. The nozzle diameter has been 38 mm and the spray velocity at the nozzle 0.3 m/s

The crude reaction product fed into the settler had a catalyst concentration of 1 to 20 wt%. The product stream taken off the settler had a catalyst concentration of 0.1 wt% with an average catalyst size of 5 µm. In the conical bottom part of the settler a catalyst layer accumulates with an average thickness increasing speed of 0.2 mm/month.

### Example 3

In the setup of example 1, the settler has been exchanged by the settler of example 2. Thus in this setup hydrocyclones and a settler with nozzles have been used.

The crude reaction product fed into the hydrocyclones had a catalyst concentration in the range from 1 to 10 wt%. The product stream taken off the settler had a catalyst concentration of 0.01 wt% with an average catalyst size of 1 µm. In the conical bottom part of the settler almost no accumulation of catalyst has been observed over a period of one year.

### List of reference numerals

- 1: nitro compound
- 3: hydrogen
- 5: solid catalyst
- 7: reactor
- 9: crude reaction product
- 11: hydrocyclone
- 13: stream depleted of catalyst
- 15: stream enriched in catalyst
- 17: settler
- 19: conical bottom part
- 21: catalyst comprising stream
- 23: first partial stream
- 25: second partial stream
- 27: nozzle
- 29: product stream
- 31: cooler
- 33: catalyst discharge system
- 35: stream substantially free of catalyst
- 37: stream substantially consisting of catalyst
- 39: first gas outlet
- 41: return line
- 43: third gas outlet

## Claims

1. A process for removing a heterogeneous catalyst from a crude reaction product (9) which comprises a liquid phase and a particulate catalyst, comprising at least one of the following steps:
(a) feeding the crude reaction product (9) into a hydrocyclone (11) in which at least part of the particulate catalyst is removed and taking off a product stream (13) depleted in catalyst and a stream (15) enriched in catalyst;
(b) feeding the crude reaction product (9) or, when step (a) also is carried out, the product stream (13) depleted in catalyst of step (a) into a settler (17), the settler (17) comprising a conical bottom part (19) wherein nozzles (27) are arranged in the conical bottom part (19) such that by a stream fed through the nozzles (27) a swirl is generated in the conical bottom part (19) of the settler (17), and taking off a product stream (29) and a catalyst comprising stream (21).

2. The process according to claim 1, wherein the catalyst comprising stream (21) of step (b) is taken off at the conical bottom part (19) of the settler.

3. The process according to claim 1 or 2, wherein a part of the catalyst comprising stream (21) which is taken off the settler (17) is fed into the settler (17) through the nozzles (27).

4. The process according to claim 3, wherein the part of the catalyst comprising stream (21) which is not fed into the settler (17) through the nozzles (27) is fed into a reactor (7) from which the crude reaction product (9) results.

5. The process according to any of claims 1 to 4, wherein a part of the stream (15) enriched in catalyst which is taken off the hydrocyclone (11) is recycled into the reactor (7).

6. The process according to any of claims 1 to 5, wherein the stream (15) enriched in catalyst which is taken off the hydrocyclone (11) is fed into a catalyst discharge system (33) from which a stream (37) comprising substantially catalyst and a stream (35) being essentially free of catalyst are taken off.

7. The process according to claim 6, wherein in the catalyst discharge system (33) is a filter system or a magnetic separator.

8. The process according to claim 6 or 7, wherein the stream (35) being essentially free of catalyst is fed back into the reactor (7) from which the crude reaction product (9) results.

9. The process according to any of claims 1 to 8, wherein the liquid phase of the crude reaction product (9) comprises an aromatic amine.

10. The process according to claim 9, wherein the aromatic amine is toluenediamine or aniline.

11. The process according to any of claims 1 to 10, wherein gas which may be comprised in the crude reaction product (9) is removed in the hydrocyclone (11) and/or in the settler (17).

12. A process for producing an aromatic amine, comprising following steps:
(i) feeding of a nitro compound (1), hydrogen (3) and catalyst (5) as starting materials into a reactor (7);
(ii) reacting the nitro compound and the hydrogen in presence of the catalyst forming the aromatic amine;
(iii) taking off a crude reaction product (9) comprising aromatic amine and catalyst from the reactor (7);
(iv) feeding the crude reaction product (9) into a process as claimed in any of claims 1 to 10.

13. The process according to claim 12, wherein the catalyst is Raney nickel and/or a supported catalyst based on nickel, palladium and/or platinum.

14. The process according to claim 13, wherein the catalyst is a platinum/iron catalyst supported on carbon.

15. The process according to any of claims 12 to 14, wherein the reactor (7) is a loop reactor.
